# EUROPEAN PATENT APPLICATION

(11) **EP 4 477 090 A1**
(43) Date of publication of application: **18.12.2024**
(21) Application number: 23179055.1
(22) Date of filing: 13.06.2023
(51) Int. Cl.: A23L 3/3526, A61L 2/18, B65B 55/10, A23L 3/3589

(54) **METHOD FOR CONTROLLING MICROBIOLOGICAL GROWTH IN STERILIZER AND PASTEURIZER APPLICATIONS USING MONOCHLORAMINE**

(71) Applicant: Solenis Technologies Cayman, L.P., 8200 Schaffhausen (CH)
(72) Inventor: Palladini, Aldo, 8200 Schaffhausen (CH); Koehorst, Toine, 8200 Schaffhausen (CH); Moering, Christina, 8200 Schaffhausen (CH)
(74) Representative: LKGlobal UK Ltd.

(57) **Abstract**

The present invention relates to a process for controlling microbiological growth in a sterilizer or pasteurizer using monochloramine, wherein the monochloramine is contained in a process liquid of the sterilizer or pasteurizer. The present invention further relates to the use of monochloramine for controlling microbiological growth in a sterilizer or pasteurizer.

## Description

### Technical Field

The present invention relates to a process for controlling microbiological growth in a sterilizer or pasteurizer using monochloramine, wherein the monochloramine is contained in a process liquid of the sterilizer or pasteurizer. The present invention further relates to the use of monochloramine for controlling microbiological growth in a sterilizer or pasteurizer.

### Technical Background

Sterilizers and pasteurizers are widely used devices to sterilize and pasteurize, respectively, products of all kind. The are particularly useful in the food and beverage industry to sterilize and pasteurize, respectively, food and beverages which may be packed in food containers of all kind, such as cans, bottles or other closed containers of basically any shape, and which may be made from a variety of materials, such as in particular metal, glas or plastics, or may even be paper-based.

Sterilization is any process which kills, removes, or deactivates all forms of microorganisms, such as spores, fungi, bacteria, and unicellular eukaryotic organisms and other biological agents in a particular object or the surface thereof. After sterilization, an object is sterile or aseptic, i.e. there are no more any microorganism in or on the object. Sterilization can be performed using various techniques, such as heating the object to be sterilized at a certain temperature above 100°C, exposure to certain chemicals, or irradiation, in each case for a certain appropriate period of time. In the sterilization of food and beverage most commonly heating technologies are used, in particular, if the food or beverage is already packaged in metal cans, glassware, such as bottles, jars or pots, or plastic containers. Sterilization may for instance be expressed in sterilization units (SU) for *clostridium botulinum* which is the common reference for sterilization, wherein 1 SU means that an object to be sterilized was held for one minute at 121,1 °C. A known sterilization process is for instance described in US 2,733,651 A.

Pasteurization is another process of preserving food and beverages. During pasteurization, food and beverages which may already be packaged or still be non-packaged are treated with mild heat, usually to less than 100 °C, to eliminate microorganism or at least to reduce them as good as possible, and, as a consequence, to extend the shelf life of the food and beverages. The pasteurization process is intended to destroy or at least to reduce the number of microorganisms and enzymes that contribute to food spoilage or risk of disease. The reduced temperature used in pasteurization processes compared to sterilization processes ensures that there will be on the one hand a minimum effect on physical stability and flavor of the object to be pasteurized, and on the other hand a maximum extension of biological stability. However, in view of that lower temperatures are used as would be necessary for complete sterilization, some microorganism or biological agents, such as bacterial spores, may survive pasteurization. These survived microorganisms and biological agents may then contribute to faster spoilage of pasteurized food compared to sterilized food. Hence, the process of pasteurization is actually a delicate balancing of pasteurization time and temperature for a given food or beverage to achieve e desired value of pasteurization units (PU) without "over-pasteurization" or "over cooking" which may damage taste properties of the food or beverage. The specific PU varies according to the product which is treated. However, it is usually heating the product for 1 minute at a temperature of around 60°C. A known pasteurization process is for instance described in EP 0169361 B1.

Many different types of sterilizers and pasteurizer are known, such as for instance described in US 9,578,894 B2, EP 0790781 B1, and EP 0169361 B1. It is common to all these types of sterilizers and pasteurizer that they contain multiple zones of different temperatures to ensure continuous heating, treating, and cooling of any object to be sterilized or pasteurized. There is at least one first zone, usually two or more first zones, wherein first the object to be sterilized or pasteurized is stepwise heated, then there is a second zone which is a treatment zone, i.e. the sterilization zone or pasteurization zone, respectively, and after the treatment zone there is at least one third zone, usually two or more third zones for stepwise cooling. In the heating, treatment and cooling zones usually a process liquid is used for optimum heat transfer which for food or beverage applications is commonly an aqueous solution.

However, sterilizers and pasteurizers, in particular those used in the food and beverage industry require frequent disinfection due to variable temperatures in particular in the heating and cooling zones which are ideal for microbiological growth and broken bottles and cans even additionally providing nutrients for microorganism of all kind, in particular for bacteria. The microbiological growth under such optimum growth conditions quickly results in a biofilm of considerable thickness which sticks to any part of the sterilizer or pasteurizer which comes into direct contact with the process liquid. Moreover, biofilm may even occur on parts of the sterilizer or pasteurizer which do not directly come into contact with the process liquid but maybe only indirectly, for instance by vapor, such as water vapor, produced from the heated process fluid. The required disinfection is typically performed using so-called "boil outs" during which the whole sterilizer or pasteurizer including the heating and cooling zones is heated for a certain time to sterilizing temperatures in order to kill all microorganism. In addition, if already a considerable amount of biofilm has been formed, mechanical cleaning may become necessary. Such frequent boil-outs which may even require mechanical cleaning are undesirable as they are laborious, time-consuming and, consequently, economically and environmentally unfavorable.

The main reasons for the required frequent disinfection of sterilizers and in particular pasteurizers are to ensure low microbiological counts, especially of harmful species such as e. *coli,* no impact on the visual appearance of the object to be disinfected or pasteurized, in particular cans and bottles, such as corrosion, staining, pitting, and blackening, prevention of fouling and buildup of biofilm in the sterilizer or pasteurizer decreasing the sterilization efficacy and pasteurization efficacy, respectively, and safety in handling and application of biocides.

In order to control or at least to suppress microbiocidal growth in liquid systems, such as aqueous solutions, typically organic biocides or strong oxidizing biocides are used which however give rise to several drawbacks, such as reduced efficiency, lower safety, higher corrosion risk, high consumption of biocide and higher overall environmental impact.

Many different organic biocides have been proposed for use in controlling microbiological growths in process fluids of sterilizers and pasteurizers, such as isothiazolinones, 2,2-dibromo-3-nitrilopropionamide (DBNPA), 1-Bromo-3-chloro-5,5-dimethylhydantoin (BCDMH), bronopol, and glutaraldehyde. However, these biocides are difficult to measure and control in concentration, are effective in narrow pH ranges only and raise environmental and safety concerns due to their toxicity and being potentially allergenic or a sensitizer.

Strong oxidizing biocides, such as sodium hypochlorite, peracetic acid, hydrogen peroxide, bromine, bromine-chlorine compositions or chlorine dioxide have been described as alternative biocides. However, such types of strongly oxidizing biocides are very corrosive, are demanded fast which leads to higher consumption, react with organic compounds in the water thus lowering the effectiveness and also potentially creating undesirable disinfection byproducts, such as adsorbable organic halides (AOX). Especially their corrosiveness against metals, and hence in particular cans, leading for instance to corrosion, blackening, and stains will influence visual appearance in particular of a metal container, such as a can, after pasteurization negatively and may even cause delayed corrosion and breaking of the metal container. Additionally, it has been found that fouling and biofilm removal properties of most oxidizing biocides is limited.

Hence, there is a need for improved processes for controlling microbiological growth in sterilizers or pasteurizers which use process liquids such as aqueous solutions. Such improved processes should in particular allow effective control of microbiological growth for a broad spectrum of bacteria, moulds, and spore formers of any type. Moreover, it would be desirable if such improved processes are highly efficient even under high organic load conditions so that only a low demand of chemicals, such as biocides, is required, and that they are further highly efficient at variable conditions for temperature, pH, and water quality. Even further, it would be desirable if such improved processes do not require mixtures of different biocides, i.e. that only one biocide is sufficient to achieve the desired microbiological growth control. Even further, it would be desirable if such improved processes lead to reduced corrosion of any metal, glass and/or plastic part of the sterilizers or pasteurizers as well as of a food or beverage container to be sterilized or pasteurized. Even further, it would be desirable if such improved processes lead to reduced biofilm buildup and improved biofilm release of already existing biofilm on any part of the sterilizers or pasteurizers as well as of a food or beverage container to be sterilized or pasteurized. Even further, it would be desirable if such improved processes lead to an improved heat transfer during disinfection in order to achieve an overall reduced energy consumption. Even further, it would be desirable if such improved processes lead to a reduced number of boil-outs which are required to clean the sterilizers or pasteurizers, a generally better cleanability of fouling in sterilizers or pasteurizers and during any still required boil-out of the sterilizers or pasteurizers, to a reduced chemical consumption, reduced energy consumption, and reduced labor work. Even further, it would be desirable if such improved processes are in general environmentally friendly, for instance by having a reduced carbon footprint due to reduced chemical usage and type of products used, and that such improved processes do not form undesirable disinfection byproducts, such as AOX.

### Summary of the Invention

The present invention provides a process for controlling microbiological growth in a sterilizer or pasteurizer using monochloramine, wherein the monochloramine is contained in a process liquid of the sterilizer or pasteurizer.

The invention is further directed to the use of monochloramine for controlling microbiological growth in a sterilizer or pasteurizer.

### Description of Embodiments

It has surprisingly been found that the above problems can be overcome with a process for controlling microbiological growth in a sterilizer or pasteurizer using monochloramine, wherein the monochloramine is contained in a process liquid of the sterilizer or pasteurizer.

The process of the present invention can be employed in any known type of sterilizer or pasteurizer which uses a process liquid. Sterilizer or pasteurizer which use a process liquid are known by the skilled person and can be readily selected based on the object to be disinfected or pasteurized, respectively, taking for instance into account the properties of any potential containers wherein the object to be disinfected or pasteurized is contained, such as material and dimensions thereof, and the job size. For instance, in particular in the field of food and beverage disinfection, suitable types of sterilizers are continuous sterilizers, such as rotary cooker and coolers, such as for instance commercially available from John Bean Technologies Corporation (JBT), or hydrostatic sterilizers, such as for instance commercially available from John Bean Technologies Corporation (JBT) and STORCK, as well as discontinuous sterilizers, such as static retorts, crateless retorts, barriquand steriflows, or stock rotomats. Similar devices or slightly modified devices can in principle also be used for pasteurization. In such case, the process is merely driven at a lower temperature so that the process is a pasteurizing process. One particularly suitable type of a pasteurizer is for instance a tunnel pasteurizer. In the process of the present invention, continuous sterilizers or pasteurizers are particularly preferred. Most preferred are pasteurizer, and in particular continuous pasteurizers, such as a tunnel pasteurizer.

It is further preferred that the sterilizer or pasteurizer is for sterilization or pasteurization, respectively, of food or beverages. The food or beverage may be contained in any suitable container, such as cans, bottles, jars, pots, or trays, which may be made from any suitable material, such as metal, glass, plastics or paper, or combinations thereof. It is preferred that the object to be sterilized or pasteurized, for instance food or beverages, is contained in metal or glass containers, in particular in cans. The metal containers may be preferably made from steel, iron, or aluminum. Particularly preferred is that the object to be sterilized or pasteurized is an aluminum can which may contain food or beverages.

In a preferred embodiment of the process of the present invention, the microbiological growth in a sterilizer is controlled, and the sterilizer is preferably configured for sterilizing food or beverage containers, preferably cans or bottles.

In another preferred embodiment of the process of the present invention, the microbiological growth in a sterilizer is controlled, and the sterilizer is preferably configured for sterilizing food or beverage containers, preferably cans or bottles.

The sterilizers and pasteurizers used in the process of the present invention may comprise at least one first zone, which is a heating zone, preferably two or more first zones (which independently can be regarded as sub-zones of the overall first heating zone), more preferably three or more first zones, such as exactly three first zones, and even more preferably four or more first zones, wherein first the object to be sterilized or pasteurized is preheated. If more than one first zone exists (i.e. more than one first sub-zone), heating may be performed stepwise, such as for instance stepwise increasing the temperature in each step between 5 and 50°C, such as between 10 and 40°C, between 10 and 30°C, or between 10 and 20°C. The heating in this first zone is performed using a process liquid as heat transfer medium. Usually each of the at least one first zone contains a separate process liquid which is heated to a certain temperature. Normally, the temperature in consecutive first zones stepwise increases in order to allow for continuously increasing the temperature of the object to be disinfected or pasteurized when being transported through the more than one first zones. However, modifications of such standard first zone heating are of course also possible. For instance, it is also sensible that only one process liquid is used within the first zone, even if there are more than one first zone. This means, that albeit there are at least two or more first zones, the process liquid in these first zones may be the same and the process liquid of the two or more first zones may be in liquid connection with each other.

The sterilizers and pasteurizers used in the process of the present invention further comprise after the first zone a second zone which is a treatment zone, i.e. the sterilization zone or pasteurization zone, respectively. In this zone sterilization or pasteurization, respectively, is performed. Usually, the treatment zone is one single zone, wherein the object to be sterilized or pasteurized is kept at a temperature suitable for sterilization or pasteurization, respectively. However, it is of course also sensible that the second treatment zone is divided into more than one, such as two, three, four or five sub-zones wherein the object to be sterilized or pasteurized is treated at different temperatures. In a preferred embodiment, the sterilizer or pasteurizer used in the process of the present invention has three second treatment zones. When the inventive process is performed in a sterilizer, the temperatures in the second treatment zone may be between 100 and 200°C, such as between 110 and 180°C, or 120 and 150°C, and preferably between 125 and 140°C. When the inventive process is performed in a pasteurizer, the temperatures in the second treatment zone may be between 40 and 100°C, such as between 45 and 90°C or 50 and 80°C, and preferably between 55 and 70°C. The time period of the treatment is not particularly limited. However, the time period should be sufficient to either kill all microbiological activity (in case sterilization is performed) or to sufficiently reduce the microorganism count that acceptable pasteurization is achieved (in case pasteurization is performed). For instance, the heat treatment in the second zone can last for at least one minute, or at least two minutes, or at least three minutes, or at least four minutes, or at least five minutes, or at least six minutes. Usually, the heat treatment is not longer than 20 minutes, such as not longer than 15 minutes, or not longer than 10 minutes, or not longer than 8 minutes, or not longer than 6 minutes. Temperature and time period may vary depending on the object to be sterilized or pasteurized, respectively, and can be adjusted according to needs for a particular sterilization or pasteurization job.

The sterilizers and pasteurizer used in the process of the present invention may further comprise at least one third zone, which is a cooling zone, preferably two or more third zones (which independently can be regarded as sub-zones of the overall third cooling zone), more preferably three or more third zones, such as exactly three third zones, and even more preferably four or more third zones, wherein the object to be sterilized or pasteurized is cooled after being sterilized or pasteurized, respectively. If more than one third zone exists (i.e. more than one third sub-zone), cooling may be performed stepwise, such as for instance stepwise reducing the temperature in each step between 5 and 50°C, such as between 10 and 40°C, between 10 and 30°C, or between 10 and 20°C. The cooling in this third zone is also performed using a process liquid as heat transfer medium. Usually each of the at least one third zone contains a separate process liquid which has a certain temperature. Normally, the temperature in consecutive third zones stepwise decreases in order to allow for continuously reducing the temperature of the object to be disinfected or pasteurized after disinfection or pasteurization, respectively, when being transported through the third zone. However, modifications of such standard third zone cooling are of course also possible. For instance, it is also sensible that only one process liquid is used within the third zone, even if there are more than one third zone. This means, that albeit there are at least two or more first zones, the process liquid in these third zones may be the same and the process liquid of the two or more third zones may be in liquid connection with each other.

A preferred sterilizer or pasteurizer used in the process of the present invention has two to four first heating zones, two to four second treatment zones, and two to four third cooling zones. A more preferred sterilizer or pasteurizer used in the process of the present invention has three first heating zones, three second treatment zones, and three third cooling zones. An even further preferred sterilizer or pasteurizer used in the process of the present invention is continuous sterilizer or pasteurizer which has three first heating zones, three second treatment zones, and three third cooling zones. Most preferably, in the process of the present invention a tunnel pasteurizer which has three first heating zones, three second treatment zones, and three third cooling zones is used.

The process liquid may in principle be any process liquid commonly used in sterilizers or pasteurizers, respectively, Usually, in particular if food or beverages are the objects to be sterilized or pasteurized, the process liquid in any of the first, second or third zones as described above is an aqueous solution. Such aqueous solution may be basically pure water but may also contain further additives, such as corrosion inhibitors, scale inhibitors, combined products for scale and corrosion inhibition, defoamers, dispersants, and filmers in order to increase the performance of the process liquid. It is preferred that in the process of the present invention the process liquid is an aqueous solution.

In the process of the present invention, monochloramine is contained in the process liquid, preferably in an aqueous solution which is used as the process liquid. The monochloramine acts as a biocide in the process liquid and it has surprisingly been found that the addition of monochloramine allows for effective control of microbiological growth for a broad spectrum of bacteria, moulds, and spore formers of any type. Moreover, the addition of monochloramine makes the sterilizing and pasteurizing processes highly efficient even under high organic load conditions (for instance if cans or bottles are broken and their content is spilled into the process liquid) so that only a low demand of additional chemicals, such as biocides, in particular monochloramine, is required. Moreover, the addition of monochloramine allows for the use of highly variable conditions for temperature, pH, and water quality. This is particularly surprising as biocides which are presently commonly used in sterilizing and pasteurizing processes, such as sodium hypochlorite, chlorite, peracetic acid, peroxides (in particular hydrogen peroxide, H₂O₂), sodium bromide or any derivates of bromide such as bronopol, DBNPA, BCDMH, isothiazolinones, glutaraldehyde, and quaternary ammonium compounds, show activity usually only within a rather narrow temperature and/or pH-range. Even further, the addition of monochloramine does not necessarily require mixtures of different biocides, i.e. only the monochloramine is sufficient to achieve the desired microbiological growth control. Even further, the addition of monochloramine surprisingly leads to reduced corrosion of any metal, glass and/or plastic material, which may be part of the sterilizers or pasteurizers as well as of any food or beverage container containing the food or beverage to be sterilized or pasteurized. In particular, it has been found that metal containers, such as aluminum containers, such as aluminum cans do not show any corrosion even after having been treated for one hour at various pH-values with an aqueous solution containing monochloramine. Even further, the addition of monochloramine leads to reduced biofilm buildup and improved biofilm release of already existing biofilm on any part of the sterilizers or pasteurizers as well as of an object to be sterilized or pasteurized, such as a food or beverage container. The reduction of already existing biofilm is particularly surprising as such reduction action has not been seen as particularly pronounced for any of the presently commonly used biocides. Even further, the addition of monochloramine surprisingly leads to an improved heat transfer during disinfection in order to achieve an overall reduced energy consumption. Even further, the addition of monochloramine leads to a reduced number of boil-outs which are required to clean the sterilizers or pasteurizers, a generally better cleanability of fouling in sterilizers or pasteurizers and during any still required boil-out of the sterilizers or pasteurizers, to a reduced consumption of chemicals, and reduced labor work for potential additionally required physical cleaning. Even further, the addition of monochloramine is in general environmentally friendly, because its use leads to a reduced carbon footprint due to a reduced use of chemicals (i.e. in particular reduced amount of biocide as only low amounts of monochloramine are required) as well as the addition of monochloramine does not form undesirable disinfection byproducts, such as AOX.

The monochloramine can be obtained from any suitable source. The monochloramine can be formed as a stock solution, such as an aqueous dilute form thereof, which can be introduced to the process liquid. The monochloramine can preferably be formed *in situ* in stock solution or process liquid from a monochloramine precursor. The monochloramine can be formed on-site or off-site. The monochloramine can be prepared accordingly to any suitable method. For example, monochloramine can be produced by one of the techniques described in U.S. Patent Nos. 4,038,372, 4,789,539, 6,222,071, 7,045,659, 7,070,751, and 8,632,794. The monochloramine can be formed by reacting dilute ammonia solution or at least one ammonium salt or other nitrogen source with at least one chlorine-containing oxidant. The monochloramine can be formed from a monochloramine precursor by reacting a dilute ammonia solution with sodium hypochlorite, calcium hypochlorite, or other hypochlorite source, or any combination thereof. Monochloramine can be prepared by adding an ammonia solution and sodium hypochlorite to dilution water to achieve a one to one molar ratio of ammonia and sodium hypochlorite. The monochloramine can be formed by reacting at least one ammonium salt with sodium hypochlorite or other hypochlorite source. For example, the ammonium salt can be ammonium carbamate, ammonium bromide, ammonium sulfate, ammonium hydroxide, ammonium chloride, or a combination thereof. Preferably, the monochloramine is formed by reacting ammonium carbamate with alkali hypochlorite, preferably sodium hypochlorite, as for instance described in US Patent No. 8,632,794. Monochloramine can be produced on-site at a sterilizer or pasteurizer using such reactions and be used immediately or stored before use. Monochloramine can be produced off-site and transported on-site for use.

For on-site production of monochloramine, a method and apparatus can be used for mixing at least two reactants or components, such as dilute ammonia solution, such as dilute ammonium carbamate solution, and sodium hypochlorite, to form the desired reaction product. The apparatus can comprise preparatory makedown unit equipment. The apparatus can include a reactor, a reactor system, a generator, a small-volume generator, a vessel, or an in-line mixer. The method and apparatus can be useful in controlling reactions that may be inherently dangerous, for example, wherein the mixing of the components has the potential to produce hazardous compounds or components. Precautions can be taken to ensure that the molar ratio of each reactant is precisely metered, as well as incoming makeup water if used in the reaction. As an example, the method and apparatus can be used for mixing an ammonia-containing chemical (e.g., ammonia) and a hypochlorite-containing chemical (e.g., hypochlorite), the nature of which is inherently dangerous. The mixing of an ammonia-containing chemical and a hypochlorite-containing chemical can be controlled carefully to avoid the production of hazardous compounds such as dichloramine, trichloramine, and chlorine gas.

The equipment used for mixing the at least two reactants or components, such as dilute ammonia solution and sodium hypochlorite, to form monochloramine, optionally can include an automated control mechanism. The control mechanism can relate one or more process control parameters to a monitored reaction condition, such as temperature. A differential temperature method of controlling an exothermic or endothermic chemical reaction optionally can be used. The chemical reaction can be an exothermic reaction, and the temperature difference can be a temperature increase. The chemical reaction can be an endothermic reaction, and the temperature difference is a temperature decrease. The method can include measuring a temperature of a first reactant flowing at a first flow rate, contacting the first reactant with a second reactant, and then measuring the temperature of a reaction product formed by a reaction between the first and second reactants. The temperature difference between the measured temperature of the first reactant and the measured temperature of the reaction product can be used to monitor the reaction, and adjustments can be made based on the temperature difference. The flow rate of the first reactant can be adjusted based on the temperature difference. The second reactant can be made to flow at a second flow rate, and the flow rate of the first reactant and/or the second reactant can be adjusted based on the temperature difference. The first temperature reading can optionally be right at the initial time that the reactants are brought together or some other time if desired. The second reading, used to obtain the temperature differential, can be a time where maximum temperature increase or decrease occurs from the reaction (e.g., the maximum increase from the exothermic reaction or maximum decrease from the endothermic reaction, whichever the case may be). This temperature difference from the reaction can be used to determine and/control the reaction to ensure that the reaction and the product from the reaction is the desired reaction product (e.g., monochloramine) and/or to ensure that reaction is proceeding in an efficient or correct manner.

In principle, the amount of monochloramine contained in the process liquid used in the process of the present invention is not particularly limited. For instance, the amount of monochloramine may be from 0.01 ppm to 20000 ppm, such as from 0.01 ppm to 10000 ppm, or from 0.05 ppm to 5000 ppm, or from 0.05 ppm to 1000 ppm, or from 0.05 ppm to 500 ppm, or from 0.08 ppm to 300 ppm, or from 0.1 ppm to 200 ppm. In a preferred embodiment, the amount of monochloramine is from 0.1 ppm to 100 ppm, such as from 0.2 ppm to 80 ppm, or from 0.5 ppm to 50 ppm, or from 0.7 to 30 ppm, or from 0.8 to 20 ppm, or from 1 ppm to 10 ppm. In some embodiments, the amount of monochloramine is from 2 ppm to 5 ppm, such as 3 ppm. The ppm values are expressed relative to the amount of water in the process liquid. It surprisingly has been found that even such very small amounts of monochloramine compared to significantly higher amounts of other biocides which are commonly used in sterilizers and pasteurizers already provide the above described beneficial and pronounced effects. Standard treatment amounts of currently used biocides in sterilizer and pasteurizer process liquids are around 600 to 10000 ppm. For instance, a currently used standard biocide treatment is 1000 ppm isothiazolinone, or 700 ppm bronopol.

In view of that even such considerable low amounts of monochloramine are already highly effective, another advantage of the process of the present invention is that no complex mixtures of biocides are required. Hence, in a preferred embodiment, in the process of the present invention no other biocide than monochloramine is used, i.e. the monochloramine is the only biocide in the process liquid.

The monochloramine can be added to any process liquid of the sterilizer or pasteurizer in any suitable manner at each addition point that might be considered suitable by the skilled person. The addition can be continuous, substantially continuous, intermittent, cyclic, batch, or any combination thereof. The addition can be performed at one or more stages or locations. In particular, the monochloramine is preferably added to at least one of the first and/or third zones of the sterilizer or to at least one of the first, second, or third zones of the pasteurizer, respectively. Most preferably, the monochloramine is added to at least one zone, preferably all zones, wherein the process liquid has a temperature which in principle would allow for optimum microbiological growth, i.e. such as for instance from 15 to 80°C, from 15 to 60°C, from 20 to 55°C, from 25 to 50°C, or from 30 to 40°C.

In a further preferred embodiment, the monochloramine or the monochloramine precursor is dosed to the process liquid in separate batches only every 30 minutes or longer, such as every 1 hour or longer, or every 2 hours or longer, or every 3 hours or longer, or every 4 hours or longer, or even every 6 hours or longer, preferably every 8 hours or longer, more preferably every 7 hours or longer, even more preferably every 10 hours or longer, such as every 12 hours or longer. It surprisingly has been found that monochloramine can be batch-wise dosed even over long time intervals without impairing the biocidal action of the monochloramine in a process liquid of the sterilizer or pasteurizer. This is particularly surprising as biocides which are presently commonly used in sterilizer or pasteurizer must be continuously dosed in order to achieve a constant biocidal activity. Continuous dosing, however, makes sterilizer and pasteurizer applications more complex and, hence, prone to potential operating problems and failures. In case of batch-wise adding, the amounts dosed at each batch wise addition may be the amounts and amount ranges as specified above. Alternatively, the amount of (remaining) monochloramine in the process liquid before batch-wise addition can be determined and an appropriate amount of monochloramine can then be added to reach a total amount of monochloramine in the process liquid as specified above. It is preferred that after each batch-wise addition the amount of monochloramine in the process liquid is as specified above for the amount of monochloramine. As at least some monochloramine will be continuously consumed between two batch-wise additions, the amount of monochloramine in between the two batch-wise additions will decrease and, hence, vary. However, it has been found that the biocidal activity of the monochloramine in the process fluid is surprisingly not impaired by such concentration decrease. The operator of the sterilization or pasteurization process can easily monitor the amount/concentration of monochloramine in the process liquid and can for instance set a lower threshold for the amount/concentration of monochloramine at which addition of further monochloramine is made. Suitable lower thresholds are the lower limits of the monochloramine amount as described above, such as 0.01 ppm, 0.05 ppm, 0.1 ppm, 0.2 ppm, 0.5 ppm, 1 ppm, or 2 ppm. The operator can also set an upper threshold of the monochloramine amount as described above which shall be achieved after each batch-wise addition, such as the upper limits of the monochloramine amount as described above, such as 20000 ppm, 10000 pm, 5000 ppm, 1000 ppm, 500 ppm, 100 ppm, 80 ppm, 50 ppm, 20 ppm, or 10 ppm. Measuring methods to determine the monochloramine content in a liquid are well known by the skilled person and can be readily applied according to need.

The present invention further relates to the use of monochloramine for controlling microbiological growth in a sterilizer or pasteurizer. Preferably, the formation of biofilm is controlled and/or already existing biofilm is removed. All details as set forth above in the context of the process of the present invention, for instance as regards the sterilizers or pasteurizers, the monochloramine and amounts thereof, are also equally applicable to the use of the present invention.

## Claims

1. A process for controlling microbiological growth in a sterilizer or pasteurizer using monochloramine, wherein the monochloramine is contained in a process liquid of the sterilizer or pasteurizer.

2. The process of claim 1, wherein the process liquid is an aqueous solution.

3. The process of claim 1 or 2, wherein the monochloramine is contained in the process liquid in an amount of 0.1 ppm to 1000 ppm, preferably 0.1 ppm to 500 ppm, and more preferably 0.1 ppm to 100 ppm.

4. The process of any of claims 1-3, wherein the monochloramine is contained in the process liquid in an amount of 1 ppm to 10 ppm.

5. The process of any of claims 1-4, wherein the sterilizer or pasteurizer is for sterilization or pasteurization of food or beverages.

6. The process of any of claims 1-5, wherein the microbiological growth in a sterilizer is controlled, and wherein the sterilizer is configured for sterilizing food or beverage containers, such as cans or bottles.

7. The process of any of claims 1-5, wherein the microbiological growth in a pasteurizer is controlled, and wherein the pasteurizer is configured for pasteurizing food or beverage containers, such as cans or bottles.

8. The process of any of claims 1-7, wherein no other biocide than monochloramine is used.

9. The process of any of claims 1-8, wherein is monochloramine is produced in situ from monochloramine precursors.

10. The process of claim 9, wherein the monochloramine precursor is a mixture of ammonium carbamate and alkali hypochlorite.

11. The process of any of claims 1-10, wherein the monochloramine or the monochloramine precursor is dosed to the process liquid in separate batches every 30 minutes or longer, preferably every 1 hour or longer, more preferably every 2 hours or longer, and even more preferably every 3 hours or longer.

12. The process of any of claims 1-11, wherein the monochloramine or the monochloramine precursor is dosed to the process liquid in separate batches every 6 hours or longer.

13. The process of any of claims 1-12, wherein the amount of monochloramine is continuously monitored, and monochloramine is added if the amount of monochloramine reaches a pre-defined lower threshold value for the amount of monochloramine, such as 0.1 ppm, and preferably 1 ppm, so to reach after monochloramine addition a pre-defined upper threshold value for the amount of monochloramine, such as 1000 ppm, preferably 100 ppm, and most preferably 10 ppm.

14. The use of monochloramine for controlling microbiological growth in a sterilizer or pasteurizer.

15. The use of claim 14, wherein the formation of biofilm is controlled and/or already existing biofilm is removed.
